# EUROPEAN PATENT APPLICATION

(11) **EP 1 275 384 A1**
(43) Date of publication of application: **15.01.2003**
(21) Application number: 01122360.9
(22) Date of filing: 19.09.2001
(51) Int. Cl.: A61K 31/155, A61K 31/64, A61P 3/10

(54) **Pharmaceutical compositions for oral use, containing, in combination, metformin and glyclazide**

(30) Priority: 09.07.2001 IT FI010126
(71) Applicant: Molteni L. E C. Dei Fratelli Alitti Societa' Di Esercizio S.P.A., 50018 Scandicci (IT)
(72) Inventor: Mannucci, Edoardo, 50047 Prato (IT); Angeli, Roberto, 50018 Scandicci (IT); Bonifacio, Marco, 53034 Colle Val d'Elsa (FL) (IT)
(74) Representative: Gervasi, Gemma, Dr.

(57) **Abstract**

Described herein are pharmaceutical compositions for oral use, containing, in combination, metformin and glyclazide, useful for the control of glycaemia in patients affected by diabetes type II.

## Description

### Field of the invention

The present invention relates to pharmaceutical compositions for oral use, containing, in combination, metformin and glyclazide.

### State of the art

As is known, diabetes type II is a frequent metabolic illness, characterized by insulin-resistance and by deficit of insulin secretion, with consequent hyperglycaemia.

Accurate control of glycaemia, maintaining fasting values, as well as values after meals, close to normality is important for the long-term prevention of the chronic complications of diabetes.

In the case where a proper diet together with physical exercise prove in themselves insufficient to bring and maintain glycaemia within normal values, it is necessary to intervene on the patient with the aid of appropriate drugs.

The drugs that are commonly used in the treatment of hyperglycaemia in patients with diabetes type II can be divided into three major categories:
a) drugs that stimulate insulin secretion, such as sulphonylureas and meglitinides. As an alternative, also insulin can be administered by subcutaneous route;
b) drugs that increase insulin sensitivity (metformin or thiazolidinediones); and
c) drugs that slow down intestinal absorption of carbohydrates (acarbose).

The main scientific societies recommend starting treatment with an oral drug (preferably a sulphonylurea in patients of normal weight and metformin in overweight patients); in the cases in which just one drug is not sufficient to maintain an adequate control of glycaemia, a second drug is added, reserving the treatment with insulin to the cases not adequately controlled with a combination of two oral drugs.

For the control of glycaemia various drugs belonging to the classes indicated above have been used, such as metformin, glybenclamide, chlorpropamide, fenformin and glyclazide.

There are moreover known compositions containing two of the aforesaid active principles in combination.

Among the latter, the one most extensively studied is the association of glybenclamide and metformin; on the other hand, some studies indicate that the combination of glybenclamide and metformin could increase long-term cardiovascular mortality as compared to therapy with just one drug.

Also known are pre-constituted combinations of chlorpropamide and metformin. Chlorpropamide, which is a first-generation sulphonylurea, induces, however, water retention and phenomena of intolerance to alcohol (antabuse effects), for which reason it is not considered a first-choice sulphonylurea. Finally, there are available combinations of glybenclamide (or chlorpropamide) with fenformin, which is another biguanide having an efficacy similar to that of metformin. The use of fenformin is, however, not recommended on account of the high incidence of lactic acidosis, which constitutes a serious and frequently fatal adverse effect.

Compositions containing metformin and glyclazide have, instead, never yet been described.

Both metformin and glyclazide are commonly used in monotherapy for the treatment of patients with diabetes type II in whom the non-pharmacological therapies (diet and physical exercise) alone are not sufficient to maintain a good metabolic control. Metformin, which is preferred in patients who are overweight, is currently the hypoglycaemic drug most widely used in the world and is generally used at doses of 1500-2500 mg/day. Glyclazide, which is the sulphonylurea most widely used in Europe, is instead administered at doses of 80-320 mg/day. When metformin alone, or glyclazide alone, at the highest doses (2000 mg/day and over of metformin, or else 240 mg/day and over of glyclazide) are not sufficient to maintain a good metabolic control, the two drugs can be combined, with extempore associations. In this case, however, the number of tablets to be taken is rather high.

From what has been said above, there emerges clearly the interest in developing new pharmaceutical compositions useful for the control of glycaemia in patients who suffer from diabetes type II.

### Detailed description of the invention

It has now been surprisingly found, and forms the subject of the present invention, that pharmaceutical compositions for oral use containing as active principles metformin and glyclazide are useful in the treatment of diabetes type II in patients not adequately controlled with metformin alone (starting from the lowest dosages of glyclazide) or with a sulphonylurea alone (with the highest dosage of glyclazide).

Pharmaceutical compositions according to the invention present considerable advantages as compared to active principles administered in a separate form or to other known pharmaceutical compositions, whilst eliminating the drawbacks presented by formulations described in the prior art described above.

In the first place, all of the present formulations enable an improvement in patient compliance, with a reduction in the number of tablets that need to be taken.

It is known that the compliance of patients to prescriptions of multiple pharmacological treatments to be taken over prolonged periods is only modest, above all when the number of tablets is high. On the other hand, diabetes mellitus type II often requires treatment with more than one active principle. In addition, amongst type II diabetics the prevalence of other disorders associated to insulin-resistance (hypertension, alterations of the lipid pattern, etc.), which frequently require additional pharmacological forms of treatment, is high. The use of tablets containing more than one active principle is thus important for improving compliance.

In addition, the combination of the two above-mentioned principles in a single formulation enables a maximum effect to be obtained with doses of each drug lower than the maximum ones envisaged in monotherapy. In fact, glyclazide presents a dose-response relation (and is approved for use) up to the dose of 320 mg/day; metformin is approved for use up to the dose of 3 g/day and presents a clear dose-response relation up to the dose of 2 g/day; the glyclazide-metformin association shows a maximum effect at the dose glyclazide 240 mg/day + metformin 1.5 g/day.

Moreover, studies currently in progress point to the fact that, in addition to the proven anti-hyperglycaemic properties, the compositions according to the invention can contribute to the prevention of chronic, micro- and macro-vascular, complications of diabetes type II.

Since the haemovascular effects of glyclazide and metformin are expressed on different phases in the process of thrombogenesis and fibrinolysis, it is highly probable that there exists a synergetic effect due to the simultaneous administration of the two active principles. Consequently, the combination of the two molecules could specifically prevent the complications of diabetes type II to an extent greater than what might be expected on the basis of the reduction of glycaemia alone.

The pharmaceutical compositions according to the invention preferably contain metformin in an amount between 250 and 1000 mg and glyclazide in an amount between 5 and 120 mg.

Preferred compositions are the ones containing 500 mg of metformin together with glyclazide in an amount between 10 and 100 mg.

Particularly preferred are the compositions according to the invention containing 500 mg of metformin in association with 20, 40 or 80 mg of glyclazide.

The compositions according to the present invention can be prepared using the techniques known in pharmacopoeia for the preparation of formulations for oral use, for example: tablets, capsules, pills, chewable tablets, preparations for sublingual absorption etc.

Particularly preferred are immediate-release tablets.

The excipients normally used are the ones used as binders (e.g., pre-gelatinized starch), diluents (e.g., microcrystalline cellulose), glidants (e.g., colloidal silica), wetting agents (e.g., Tween 80), disgregating agents (e.g., sodium amidoglycolate), lubricants (e.g., magnesium stearate), and film-coating agents (e.g., opadry oy s7163).

Particularly preferred are the formulations according to the invention in the form of high-disgregation tablets which enable immediate release of the active principles.

Particularly preferred tablets of this type are 950-mg tablets which contain (in addition to two active principles in the amounts indicated above) the following additives:

| | |
|---|---|
| Microcrystalline cellulose | as required for 950 mg |
| Pre-gelatinized starch | 60 mg |
| Hydrated colloidal silica | 6 mg |
| Tween 80 | 7 mg |
| Sodium amidoglycolate | 59 mg |
| Magnesium stearate | 5 mg |
| Opadry oy s7163 | 30 mg |

The size of an industrial lot is approximately 350 000 tablets corresponding to 322 kg.

To prepare approximately 87 500 950-mg tablets (corresponding to approximately 80.5 kg of granulate) the following are loaded in a Grall Collette granulator: 43.750 kg of metformin, 17.65 kg of microcrystalline cellulose, 5.250 kg of pre-gelatinized starch, and 0.521 kg of hydrated colloidal silica. The above ingredients are mixed for 10 minutes.

Granulation is carried out with a solution consisting of 10.341 kg of water and 0.612 kg of Tween 80.

The granulate thus obtained, which has a somewhat pasty consistency due to the granulation with the water/Tween mixture, is passed through a rotary granulator provided with a basket having holes of a diameter of 3 mm, is dried in a fluid bed (RH = 1.3%) and is co-ground at 16 mesh with 5.163 kg of sodium amidoglycolate and 7.000 kg of glyclazide.

After the four sub-lots have been pooled together, the procedure continues with the addition of 1.750 kg of magnesium stearate, and finally with compression and film coating.

The compositions according to the invention can be used in the treatment of diabetes mellitus type II in patients who are not adequately controlled with metformin alone or with a sulphonylurea agent alone. In particular, patients not adequately controlled with metformin alone could pass to metformin 500 mg + glyclazide 20 mg three times a day, which guarantees an effective dose of metformin with the addition of a small dose of glyclazide for stimulating insulin secretion. If this is not sufficient, they could pass to metformin 500 mg + glyclazide 40 mg three times a day, and possibly to metformin 500 mg + glyclazide 80 mg three times a day. Patients not adequately controlled with a sulphonylurea agent alone could pass to glyclazide 80 mg + metformin 500 mg three times a day, so as to maintain a sufficiently high dose of sulphonylurea and add thereto the insulin-sensitizing action of metformin.

## Claims

1. Pharmaceutical compositions for oral use, containing as active principles metformin and glyclazide in combination with one another and possibly in combination with the customary excipients used for the preparation of compositions for oral use.

2. Compositions according to Claim 1, in which metformin is in an amount of between 250 and 1000 mg and glyclazide is in an amount of between 5 and 120 mg.

3. Compositions according to Claim 2, in which metformin is in an amount of 500 mg and glyclazide in an amount of between 10 and 100 mg.

4. Pharmaceutical compositions according to Claim 3, in which metformin is in an amount of 500 mg and glyclazide is in an amount of 20 mg.

5. Pharmaceutical compositions according to Claim 3, in which metformin is in an amount of 500 mg and glyclazide is in an amount of 40 mg.

6. Pharmaceutical compositions according to Claim 3, in which metformin is in an amount of 500 mg and glyclazide is in an amount of 80 mg.

7. Pharmaceutical compositions according to Claims 1-6, in the form of high-disgregation tablets.

8. Use of metformin and glyclazide for the preparation of pharmaceutical compositions according to Claims 1-7 for the control of glycaemia.

9. Use of metformin and glyclazide for the preparation of pharmaceutical compositions according to Claims 1-7 for the treatment of diabetes mellitus type II.

10. Fast-disgregation tablets containing the active principles in the amounts indicated in Claims 4 - 6 and the following additives:
| | |
|---|---|
| Microcrystalline cellulose | as required for 950 mg |
| Pre-gelatinized starch | 60 mg |
| Hydrated colloidal silica | 6 mg |
| Tween 80 | 7 mg |
| Sodium amidoglycolate | 59 mg |
| Magnesium stearate | 5 mg |
| Opadry oy s7163 | 30 mg |

11. A method for the treatment of patients affected by diabetes mellitus type II, in which the patient is administered tablets containing metformin 500 mg + glyclazide 20 mg, three times a day.

12. A method for the treatment of patients affected by diabetes mellitus type II, in which the patient is administered tablets containing metformin 500 mg + glyclazide 40 mg, three times a day.

13. A method for the treatment of patients affected by diabetes mellitus type II, in which the patient is administered tablets containing metformin 500 mg + glyclazide 80 mg, three times a day
